# EUROPEAN PATENT SPECIFICATION

(11) **EP 2 983 762 B1**
(45) Date of publication and mention of the grant of the patent: **13.09.2017**
(21) Application number: 14716294.5
(22) Date of filing: 08.04.2014
(51) Int. Cl.: A61M 5/315, A61M 5/20, A61M 5/31

(54) **INJECTION DEVICE**
INJEKTIONSVORRICHTUNG
DISPOSITIF DÝINJECTION

(30) Priority: 10.04.2013 EP 13163115
(43) Date of publication of application: 17.02.2016
(73) Proprietor: SANOFI, 75008 Paris (FR)
(72) Inventor: MORRIS, Anthony Paul, Coventry West Midlands CV7 7FY (GB); MARSH, William, Buckingham Buckinghamshire MK18 4HZ (GB); BUTLER, Joseph, Rugby Warwickshire CV21 4DU (GB); JONES, Matthew, Warwick Warwickshire CV34 6AU (GB)
(74) Representative: Keil & Schaafhausen Patent- und Rechtsanwälte PartGmbB
(86) International application number: PCT/EP2014/057008
(87) International publication number: WO 2014/166926

(56) References cited:
- EP-A1- 2 198 903
- WO-A1-2006/130098
- WO-A2-2009/010591
- DE-A1-102005 054 313
- US-A- 5 637 095

## Description

The present invention relates to an injection device, especially a pen type drug delivery device. The mechanism comprises a housing, a dose setting member, which during dose setting rotates relative to the housing in a first direction and which during dose dispensing rotates relative to the housing in a second opposite direction, and a limiting element for limiting the rotational movement of the dose setting member between a zero dose position and a maximum dose position.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. The present invention is not directed to so called fixed dose devices which only allow dispensing of a predefined dose without the possibility to increase or decrease the set dose.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is in general applicable for both types of devices, i.e. for disposable devices as well as for reusable devices.

These types of pen delivery devices (so named because they often resemble an enlarged fountain pen) are generally comprised of three primary elements: a cartridge section that includes a cartridge often contained within a housing or holder; a needle assembly connected to one end of the cartridge section; and a dosing section connected to the other end of the cartridge section. A cartridge (often referred to as an ampoule) typically includes a reservoir that is filled with a medication (e.g., insulin), a movable rubber type bung or stopper located at one end of the cartridge reservoir, and a top having a pierceable rubber seal located at the other, often necked-down, end. A crimped annular metal band is typically used to hold the rubber seal in place. While the cartridge housing may be typically made of plastic, cartridge reservoirs have historically been made of glass.

The needle assembly is typically a replaceable double-ended needle assembly. Before an injection, a replaceable double-ended needle assembly is attached to one end of the cartridge assembly, a dose is set, and then the set dose is administered. Such removable needle assemblies may be threaded onto, or pushed (i.e., snapped) onto the pierceable seal end of the cartridge assembly.

The dosing section or dose setting mechanism is typically the portion of the pen device that is used to set (select) a dose. During an injection, a spindle or piston rod contained within the dose setting mechanism presses against the bung or stopper of the cartridge. This force causes the medication contained within the cartridge to be injected through an attached needle assembly. After an injection, as generally recommended by most drug delivery device and/or needle assembly manufacturers and suppliers, the needle assembly is removed and discarded.

A disposable drug delivery device for selecting and dispensing a number of user variable doses of a medicament according to the present invention typically comprises a housing, a cartridge holder for receiving a cartridge, a lead screw or piston rod and means for driving the piston rod during dose dispensing. Such a disposable drug delivery device is known from WO 2004/078241 A1, wherein the cartridge holder is rigidly attached to the device housing. The piston rod, which acts on a cartridge bung, is advanced by a driver during dose dispensing. This known device is a manually driven device, where the component parts are in general disposed concentrically around a common longitudinal axis. During dose setting some component parts wind out of the housing and are pushed back into the housing during dose dispensing.

In the following, the distal end of an injection device or drive mechanism is referred to as the end where a cartridge and e.g. a needle are located, whereas the opposite end is the proximal end. A dose button may be provided at the proximal end.

A further differentiation of drug delivery device types refers to the drive mechanism: There are devices which are manually driven, e.g. by a user applying a force to an injection button like in WO 2004/078241 A1, devices which are driven by a spring or the like and devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

EP 2 198 903 A1 discloses a motor mechanism for a drug delivery device with a spring in the form of a strip of spring metal sheet attached to two spools. The spring is part of a motor mechanism of the drug delivery device, which further comprises a drive mechanism. At least a part of the drive mechanism is rotatable by the motor mechanism thereby advancing a bung for dispensing a dose from a container. A latch mechanism is provided for restricting rotation of the motor mechanism, which latch mechanism is depicted in EP 2 198 903 A1 as allowing a full 360° rotation upon actuation of a trigger and thereafter re-engaging for restricting further rotation of the motor mechanism.

It is an object of the present invention to provide an improved alternative to the above solutions. It is a further object to make the drug delivery device compact in size, preferably without components translating out of the housing during dose setting.

This object is solved by a device with the features of claim 1. According to a first embodiment of the present invention, the handheld injection device comprises a housing, a piston rod located within the housing, a drive member, which is preferably permanently coupled to the piston rod, and a power reservoir for driving the drive member. The drive member is axially movable between a dose setting position, in which the drive member is rotationally constrained to the housing, and a dose dispensing position, in which the drive member is rotationally de-coupled from the housing. The power reservoir comprises a reverse wound flat spiral spring having a first end attached to a first spool and a second end attached to a second spool, which is axially and rotationally constrained to drive member. In other words, the spring is movable together with the drive member between the dose setting position, which may be a proximal position, and the dose dispensing position, for example a distal position. The second spool is preferably an integral part of the drive member.

The drive member may comprise one or more component parts. If more than one component part forms the drive member, these parts may be rotationally coupled to each other, preferably by meshing gears, at least during dose dispensing, but preferably permanently. The definition of the drive member being axially movable includes embodiments, where only one of several component parts of the drive member is axially movable, whereas one or more further component parts of the drive member may be axially constrained.

The power reservoir may comprise a storage spool (first spool) and a torque output spool (second spool) arranged close to each other and a strip of spring sheet metal having two ends, each end attached to one of the spools. The strip of spring sheet metal is coiled on the storage spool in a relaxed state. The spring is preferably charged during manufacturing of the device by rotating the torque output spool thereby coiling the strip of spring sheet metal onto the torque output spool and bending the strip of spring sheet metal the other way round than in the relaxed state thus arriving in a charged state with the strip of spring sheet metal tending to re-coil onto the storage spool thereby generating a torque.

One characteristic of such a spring is that the torque remains relatively constant throughout the transfer of the spring from the torque output spool to the storage spool. For this reason the spring mechanism may be called a constant torque motor. This characteristic is particularly suitable for use in dispensing multiple doses of medication because it means that the first (with maximum stored energy in the spring) and last doses (with spring energy almost exhausted) will be delivered with very similar characteristics, such as injection speed and breakout force (this is the force required to overcome the static friction of a bung in the medication cartridge). This means that the spring can be designed around one operating condition, i.e. the torque required to overcome static friction and then to deliver the medication in an appropriate injection time. In a preferred embodiment the strip of spring sheet metal consists of stainless steel or spring steel.

In a preferred embodiment, the second end of the spring comprises a portion of reduced width and a free end portion having an increased width compared with the portion of reduced width. Further, the drive member may comprise a cylindrical spool portion having an axial slot and an adjacent narrow recess, which are adapted to the size of the spring for anchoring the spring in or on the drive member. Preferably, the axial length of the axial slot is larger than the width of the free end portion of the spring and the axial length of the narrow recess is larger than the width of the portion of reduced width of the spring but smaller than the width of the free end portion of the spring. This results in a robust anchorage of the spring on the drive member and allows an easy assembly of the spring to the drive member even with significant manufacturing tolerances of the spring.

Typically, the injection device further comprises a dose setting member, which may be a combined dose setting and dose display member, i.e. a dose indicator. To provide a visual indication of a set dose, numerals or markings may be provided on the dose indicator which are visible from the outside of the housing. Preferably, the dose indicator is axially constrained to the housing and rotates relative to the housing in either a first direction or a second opposite direction during dose setting (and dose correction, respectively) and rotates relative to the housing in the second opposite direction during dose dispensing.

To further improve the display of the actually set dose, a gauge element may be provided, which is at least partly visible through at least one aperture or window of the housing. The gauge element may be axially guided within the housing and may be in threaded engagement with the dose indicator such that rotation of the dose indicator causes an axial displacement of the gauge element. The gauge element may be at least partly interposed between the housing and the dose indicator. In other words, the gauge element may screen or uncover a contrast element, for example a housing part or a part of the dose indicator, as the gauge element travels axially within the housing. This provides an analogue optical dose set and dispense feedback to the user.

According to a further embodiment, the device may comprise two apertures or windows on opposite sides of the housing, with the gauge element being visible through both apertures or windows. Thus, this visual feedback is visible independent of the orientation (right-handed user or left-handed user) the device is held during dose dispensing.

This visual feedback feature, which is preferably provided in addition to a number display (for example provided by the dose indicator), gives clear feedback to the user regarding the approximate size of the dose set. The dispense speed of a spring driven injector mechanism may be higher than for a manual injector device, so it may not be possible to read the typical numerical dose display during dispense. The gauge feature provides feedback to the user during dispense regarding dispense progress without the need to read the dose number itself. In addition, this gauge display simulates a syringe action during dose set and dispense.

According to a preferred embodiment, the drug delivery device comprises a limiter mechanism defining a maximum settable dose and a minimum settable dose. Typically, the minimum settable dose is zero (0 IU of insulin formulation), such that the limiter stops the device at the end of dose dispensing. The maximum settable dose, for example 60, 80 or 120 IU of insulin formulation, may be limited to avoid overdosage. Preferably, the limits for the minimum dose and the maximum dose are provided by hard stop features. The limiter mechanism may comprise a first rotational stop on the dose indicator and a first counter stop on the gauge element, which abut in the minimum dose (zero) position, and a second rotational stop on the dose indicator and a second counter stop on the gauge element, which abut in the maximum dose position.

The housing may have an aperture or window and the gauge element may have a further aperture or window, which is positioned with respect to the aperture or window of the housing such that at least a part of the dose indicator is visible through the apertures or windows. The dose indicator may be marked with a sequence of numbers or symbols. With the dose indicator (number sleeve) located radially inwards of the gauge element, this allows that at least one of the numbers or symbols on the dose indicator is visible through the apertures or windows. In other words, the gauge element may be used to shield or cover a portion of the dose indicator and to allow view only on a limited portion of the dose indicator. This function of a precise dose indication may be in addition to the gauge element itself being suitable for identifying or indicating the actually set and/or dispensed dose as mentioned above. As the gauge element is axially displaced during dose setting and dose dispensing, a sliding window display is provided.

Dose setting may be effected by rotation of a dial grip which may be axially constrained to the housing. Further, dose dispensing may require pressing an axially displaceable dispense button. As an alternative, a combined dial grip and button may be provided, which is axially movable.

In addition or as an alternative to the display of a set dose by the dose indicator and/or the gauge element, a tactile and/or audible feedback may be generated during dose setting and/or during dose dispensing. For example, the injection device may further comprise a clicker sleeve which comprises teeth releasably engaging corresponding teeth of a further element which rotates together with dial grip during dose setting. Preferably, the clicker sleeve is rotationally constrained to the housing and is axially displaceable relative to the housing between a proximal dose setting position and a distal dose dispensing position. The clicker sleeve may shuttle axially as it rides over the corresponding teeth of the rotatable element, thus generating the non-visual feedback signal.

Further, it is preferred to indicate to a user the end of dose dispensing. The end of dose dispensing feedback may indicate that the device returned to its zero dose position, i.e. when a set dose has been at least substantially injected. Thus dose dispensing may still be in progress for example by further relaxation of the cartridge bung. The feedback is preferably a tactile and/or audible feedback, like the generation of a click sound by the pre-tensioned flexible clicker arm passing over an edge. According to a preferred embodiment, the dose indicator comprises a flexible clicker arm, which is displaceable by the clicker sleeve in a first direction and only during dose dispensing when the device reaches its minimum dose (zero) position in a second, opposite direction by a protruding section of the gauge element.

To prevent an underdosage or a malfunction, the drug delivery device may comprise a last dose protection mechanism for preventing the setting of a dose, which exceeds the amount of liquid left in a cartridge. The last dose protection mechanism may comprise a nut member located interposed between the clicker sleeve and a dial sleeve, which may be rotationally constrained to the dial grip. In a preferred embodiment, the dial sleeve rotates during dose setting and remains stationary during dose dispensing, whereas the clicker sleeve remains permanently rotationally constrained to the housing. Thus, in this embodiment, the nut member will only move during dose setting and will remain stationary with respect to these components during dose dispensing. Preferably, the nut member is threaded to the dial sleeve and splined to the clicker sleeve. As an alternative, the nut member may be threaded to the dial sleeve and may be splined to the clicker sleeve. The nut member may be a full nut or a part thereof, e.g. a half nut.

Preferably, the first spool is located concentrically with the piston rod on a first longitudinal axis, and the second spool is located on a second longitudinal axis, wherein the first longitudinal axis is parallel to and spaced from the second longitudinal axis. This reduces the overall length of the device. Further, the drive member may comprise a drive tube which is rotatable about the first longitudinal axis and a drive sleeve which is rotatable about the second longitudinal axis. In a preferred embodiment the drive sleeve is axially movable between the dose setting position, in which the drive sleeve is rotationally constrained to the housing, and the dose dispensing position, in which the drive sleeve is rotationally de-coupled from the housing. Preferably, the drive sleeve and the drive tube are permanently rotationally coupled, e.g. via meshing gears. As an alternative, the drive tube may be rotationally coupled to the drive sleeve only if the drive sleeve is in its dose dispensing position.

A clutch may be provided interposed between the dose indicator and the drive member, wherein the clutch allows relative rotational movement between the dose indicator and the drive member during dose setting and prevents relative rotational movement between the dose indicator and the drive member during dose dispensing.

The injection device may comprise a cartridge containing a medicament. Preferably, the spring is pre-tensioned to store the energy required to dispense the whole contents of the cartridge.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, , an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (So-matropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Non-limiting, exemplary embodiments of the invention will now be described with reference to the accompanying drawings, in which:
- Figure 1: shows an exploded view of an injection device according to an embodiment of the invention,
- Figure 2: shows a partly cut-away view of the limiter mechanism of Figure 1,
- Figure 3: shows a section view of the mechanism of Figure 1 in the dose setting position,
- Figure 4: shows an exploded view of the dose indicator and the sliding gauge,
- Figures 5a to c: show partly cut-away views during various steps of dose setting,
- Figures 6a to c: show side views during various steps of dose setting,
- Figure 7: shows a perspective view of the device of Figure 1,
- Figures 8a, b: show sectional views of the device of Figure 1 in the dose setting position and in the dose dispensing position,
- Figure 9a: shows a spring of the device of Figure 1 according to a first embodiment,
- Figure 9b: shows a spring of the device of Figure 1 according to a second embodiment,
- Figure 10: shows a detail of the device of Figure 1,
- Figure 11: shows a detail of the device of Figure 1,
- Figure 12: shows a detail of the device of Figure 1,
- Figures 13a, b: show side views of details of the device of Figure 1 in the dose setting position and in the dose dispensing position
- Figure 14: shows in an exploded view a detail of the device of Figure 1,
- Figures 15a to f: show a clicker of the device of Figure 1 during dialing and during dose dispensing,
- Figure 16: shows a detail of the device of Figure 1, and
- Figure 17: shows in a sectional view a detail of the device of Figure 1.

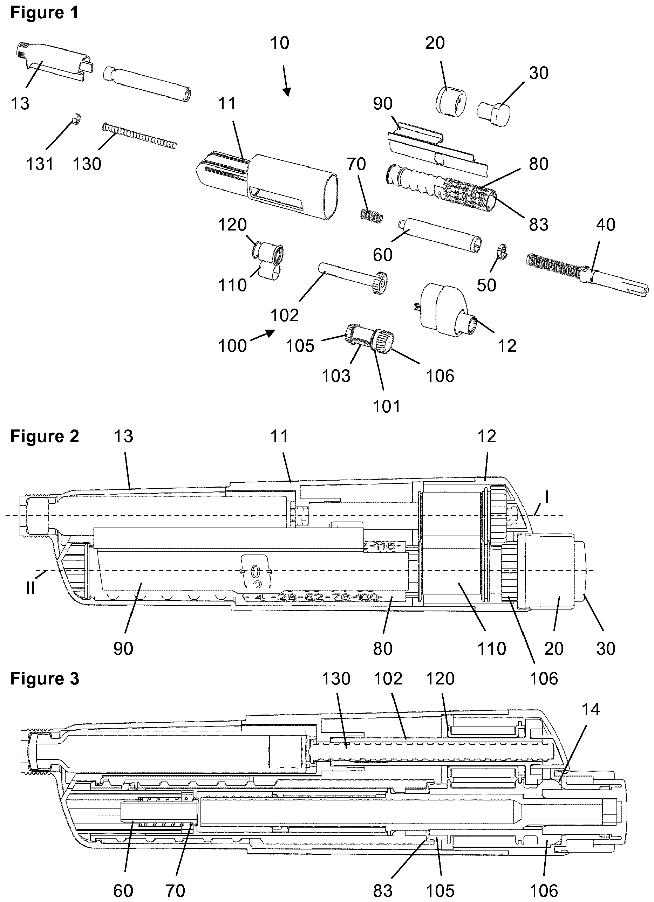

An injection device 1 according to the present invention is shown in Figure 1 in an exploded view. The injection device comprises 19 components, excluding the liquid medicament cartridge. In more detail, the device comprises a housing 10, which includes a main housing 11, a proximal cap 12 and a cartridge holder 13, a dial grip 20, a dispense button 30, a dial tube (dial sleeve) 40, a last dose nut 50, a sleeve-like clicker 60, a dispense spring 70, a dose indicator 80, a sliding gauge 90, a drive member 100 including a drive sleeve 101 and a drive tube 102, a motor spring 110, a storage spool 120, a piston rod (lead screw) 130 with a bearing 131, a lens (not shown) and a cap (not shown). Most of the components are located concentrically about one of two principle axes I and II of the mechanism as shown in Figure 2.

The operation of the device is as follows: In an "at rest" condition as shown in Figure 3 the dispense button 30 is not depressed, i.e. the device is in its dose setting position/state which allows dose setting and dose correction. In this state, the dial grip 20 is splined to the dispense button 30 and axially constrained to the housing 10 (via proximal cap 12), the dispense button 30 is rigidly coupled to the dial tube 40, the dial tube 40 is splined to the dose indicator 80, the sliding gauge 90 is threaded to the dose indicator 80 and splined to the housing 10, the clicker 60 is splined to the housing 10 and the drive sleeve 101 is splined to the housing 10, too. As can be seen in Figure 1, the drive sleeve 101 has a ring of distal teeth 105 and a ring of proximal teeth 106. Proximal teeth 106 engage with corresponding inner teeth 14 of the proximal cap 12 during dose setting. Distal teeth 105 form a releasable clutch with corresponding inner splines 83 of the dose indicator 80. This clutch is decoupled during dose setting.

Dose setting requires rotation of the dial grip 20, which causes the dose indicator 80 to rotate, as the components are operably coupled during dialing via a sequence of splined connections between the dial grip 20, dispense button 30, dial tube 40 and dose indicator 80. There is a profiled axial interface between the clicker 60 and dial tube 40, which are forced into contact by the dispense spring 70, which generates the detented dose positions of the dial grip 20 and dose indicator 80 and provides tactile and audible user feedback. The sliding gauge 90, which is threadedly engaged with the dose indicator 80, traverses axially within the housing 10 as the dose indicator 80 rotates. The sliding gauge 90 is able to traverse between rotational abutments which occur between the sliding gauge 90 and the dose indicator 80, that correspond to zero unit and max dose positions of the device. The drive sleeve 101 is rotationally restrained during dialing via a splined interface to the housing (proximal cap 12), such that, via the drive tube 102, the piston rod 130 is rotationally and axially constrained to the housing 10. The splined interface between the drive sleeve 101 and the proximal cap 12 prevents the drive sleeve 101 from rotating, during setting of a dose, under the permanently applied torque from the motor spring 110.

Rotation of the dose indicator 80 generates axial motion of the sliding gauge 90, relative to the housing 10, via its threaded engagement with the dose indicator 80 and its rotational constraint, via splines, to the main housing 11. Axial motion of the sliding gauge 90 is constrained in both the distal and proximal directions by abutments of the end face of the thread on the sliding gauge 90 with the abutment surfaces in the recessed threadform on the dose indicator 80.

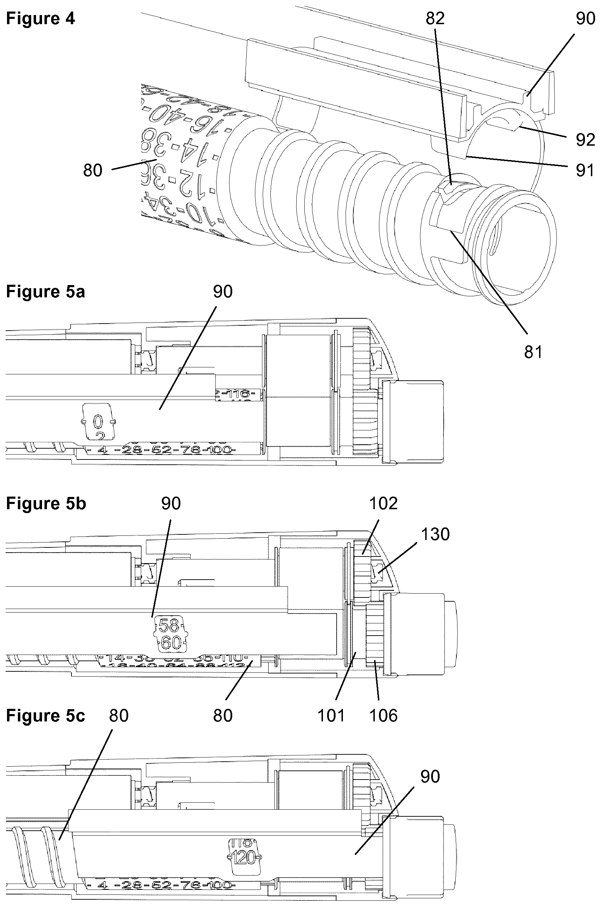

There are zero and maximum dose stops, which are generated by abutments between the sliding gauge 90 and the dose indicator 80. A section of helically formed thread on the sliding gauge 90 interfaces with a recessed threadform in the outer cylindrical surface of the dose indicator 80. This recessed threadform is limited at either end by an abutment surface. Figure 4 shows the zero unit abutment surface 81 and the corresponding stop surfaces 91 (zero dose) and 92 (maximum dose) on the sliding gauge 90. The maximum dose stop surface on the opposite end of the threadform on dose indicator 80 is not visible in Figure 4. In the zero unit position the sliding gauge 90 is at its extreme distal position. The sliding gauge 90 moves in the proximal direction as a dose is dialed up by the user, until a similar abutment occurs corresponding to the maximum dose position of the device.

At any time during dose setting and dose dispensing, the dose position of the device is displayed to the user through an aperture in the sliding gauge 90. Dose position markings are applied to the dose indicator 80 in a helical pattern, the pitch of the helical pattern of numbers matches the helical pitch of the threaded interface between the dose indicator 80 and the sliding gauge 90. For each dose position, a specific portion of the helical pattern of numbers is visible through the aperture in the sliding gauge 90 as shown in Figures 5a to 5c. The aperture in the sliding gauge 90 aligns to a larger aperture in the main housing 11. The sliding gauge 90 extends in the proximal and distal directions, either side of the aperture, for a sufficient length, such that the sliding gauge 90 obscures the helical pattern of numbers on the dose indicator 80 which would otherwise be visible through the larger aperture in the housing. The travel of the sliding gauge 90 is defined by the pitch of the threaded interface with the dose indicator 80. The larger aperture in the main housing 11 therefore makes visible to the user the surface of the sliding gauge 90 and the aperture of the sliding gauge 90 through which a portion of the helical pattern of numbers is visible. The larger aperture in the housing may be covered by a transparent lens element, which may provide magnification of the number display.

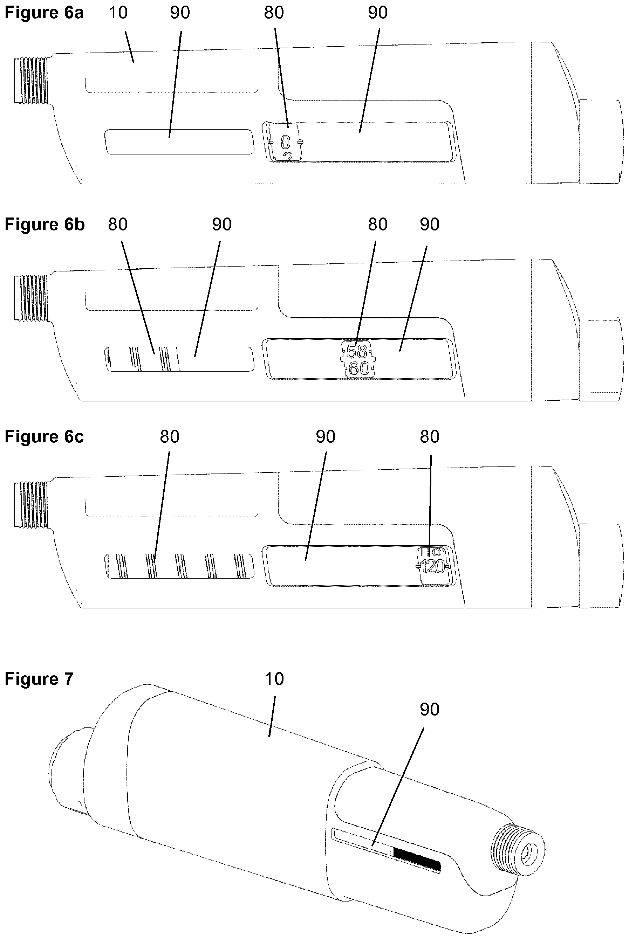

In the embodiment depicted in the Figures, the axial motion of the sliding gauge 90 is utilized to provide additional user feedback of the dose position of the device, which is considered particularly beneficial during dispensing of a dose. As shown in Figures 6a to 6c and 7 additional gauge apertures (slots) are provided in the main housing 11, through which a surface of the sliding gauge 90 is visible. As the sliding gauge 90 moves in the proximal direction, as a dose is dialed, the distal end of the sliding gauge 90 moves within the gauge apertures, uncovering a further surface of the device. This further surface may be a stationary element of the device, which is rigidly constrained to the housing 10. In an alternative embodiment this further surface may be a moving element of the device, such as the cylindrical surface of the dose indicator 80. The further surface may be colored to indicate a specific type of medicament, it may have markings or graphics to improve communication of dialing or dispensing progress to the user.

The length of the further surface which is uncovered, and therefore visible, by the proximal movement of the sliding gauge 90 is proportional to the dose position of the device, and therefore the size of the dose currently dialed. The visible further surface therefore provides an analog indication, or analog gauge, of the size of the dose dialed. The length of the analog gauge is defined by the pitch of the threaded interface between the dose indicator 80 and the sliding gauge 90, and therefore is independent from the mechanism interfaces within the device related to dose delivery. The analog gauge can therefore show greater axial movement for each dose increment that the required axial movement of the piston rod. The gauge apertures in the housing can be positioned independently from the number display aperture.

In particular, the apertures may be located on the main housing 11 in a location which is visible to the user during dispense of a dose. This may be close to the distal end of the device. Particularly this may be a location in which the number display of the dose indicator 80 could not feasibly be located. There may also be a plurality of gauge apertures. In particular there may be two gauge apertures, located on opposite sides of the device as shown in Figures 6a to 6c and 7. This increases the visibility of the analog gauge feature for users with a preference for left handed operation, or those users with a preference to hold the device with an alternative grip.

The analog gauge is particularly beneficial as an indicator of the dose position of the device during dispense of a dose. During dispense of a dose the number digit display may be changing too quickly for individual dose position markings to be legible. It may therefore be difficult for the user to understand the rate at which the dose is being dispensed, and the amount of medicament still to be dispensed. The axial motion of the analog gauge, which increasingly covers the further surface as a dose is dispensed, gives a simple visible indicator of the dispense rate and the amount of medicament still be to dispensed during the dispense event.

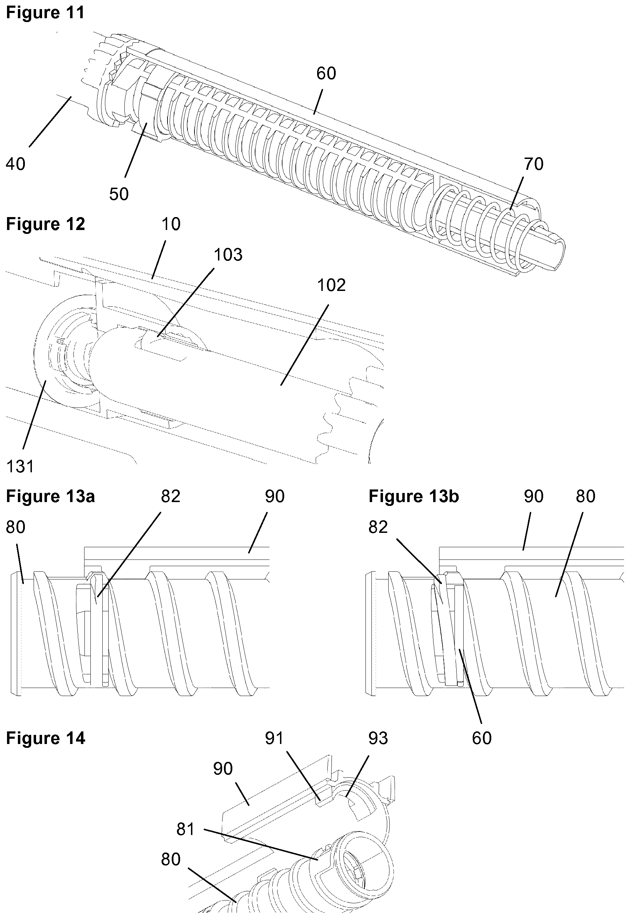

Feedback during dose setting is provided by an interaction between the dial tube 40 and the clicker 60. The clicker 60 is splined to the housing 10, and the dispense spring 70 forces the clicker 60 into axial engagement with the dial tube 40. As shown in Figure 11, there is an axial toothed interface between the components and the clicker 60 shuttles axially as the dial tube 40 is rotated, providing detented orientations. The dial tube 40 is rigidly coupled to the dispense button 30, which is splined to the dial grip 20.

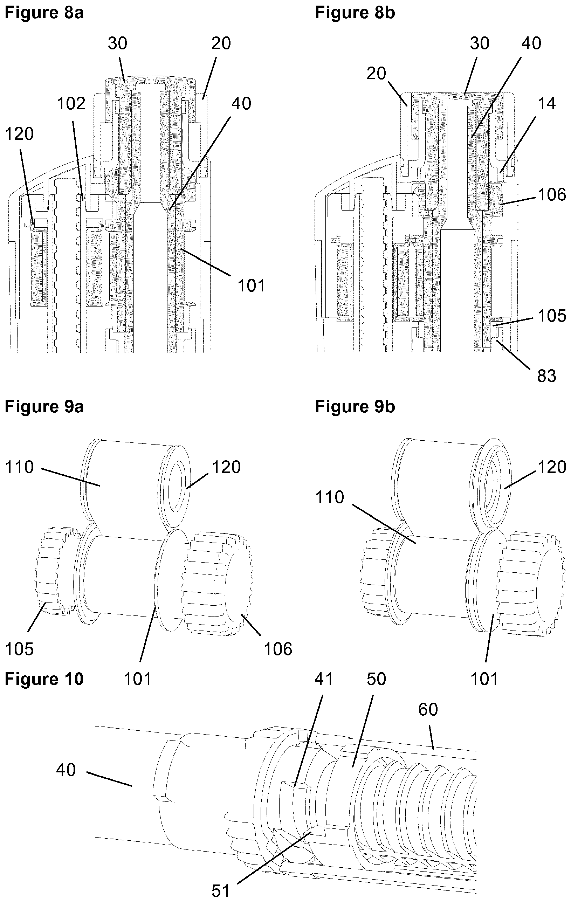

With a dose set as described above, it is possible to reduce (cancel) the set dose without dispensing by rotating dial grip 20 in the opposite direction. The reduced dose is indicated by the displays as mentioned above. Further, with a dose set, dispensing of the dose may be initiated by pressing the dispense button 30 by the user, which displaces the button in the distal direction. Figures 8a and 8b show the proximal end of the device with button 30 released, i.e. in the dose setting position, (Figure 8a) and with button 30 depressed, i.e. in the dose dispensing position, (Figure 8b). The following components are axially displaced during dispensing: dispense button 30, dial tube 40, last dose nut 50, clicker 60, drive sleeve 101, motor spring 110 and storage spool 120.

In an alternative embodiment, the dial grip and dispense button may be combined such that the dial grip is also displaced axially during dispensing.

Spline teeth on the dispense button 30 engage with corresponding spline teeth on the proximal cap 12, which forms part of the main housing 11, constraining rotation of the dispense button 30 during dispensing. The dial tube 40, which is rigidly coupled to the dispense button 30, is displaced axially such that it disengages its splined interface with the dose indicator 80. The clicker 60 is also displaced axially, which compresses the dispense spring 70. The axial force applied by the user is reacted by the dispense spring 70, and by an axial abutment between the dispense button 30 and the proximal cap 12. The dial grip 20 remains splined to the dispense button 30, and is therefore rotationally constrained. As the dial grip 20 is rotationally constrained and is decoupled from the dose indicator 80 during dispensing, the user is unable to input abuse torques to the dispensing mechanism or mistakenly adjust the dose.

By pressing button 30, the drive sleeve 101, which is axially located by the dispense button 30 and the dial tube 40 is moved axially so that it first engages spline features 105, 83 with the dose indicator 80, i.e. engaging clutch 105, 83, then disengages from its splined interface 106, 14 with the housing 10. The motor spring 110, which is wrapped around a cylindrical surface of the drive sleeve 101, is also moved axially. In the embodiment shown in Figure 9b, a radial flange on the storage spool 120 engages with a circumferential groove on the drive sleeve 101, such that there relative axial position is constrained. In the alternative embodiment of Figure 9a, the material of the motor spring 110 transmits the axial motion to the storage spool 120 so that there is no direct axial interface between the drive sleeve 101 and storage spool 120. Once the drive sleeve 101 is axially displaced it is no longer rotationally constrained, and is able to rotate under the action of the motor spring 110.

Via the geared interface between the drive sleeve 101 and the drive tube 102, the drive tube 102 is rotated which then drives the piston rod 130 through the housing into the cartridge and against a bung located therein, dispensing liquid medicament from the cartridge. During dispensing, the splined engagement between the drive sleeve 101 and the dose indicator 80, created by axial displacement of the drive sleeve 101, causes the dose indicator 80 to rotate back towards the zero unit position.

Key interfaces during dispensing are: the dispense button 30 is axially displaced by the user, creating a splined engagement with the housing 10, the dial tube 40 disengages from its splined engagement with the dose indicator 80, the drive sleeve 101 is axially displaced, creating a splined engagement with the dose indicator 80, and disengaging its splined engagement with the housing, the geared interface between the drive sleeve 101 and the drive tube 102 remains engaged (however, the amount of overlap between the gear teeth increases as the drive sleeve 101 moves axial, whilst the drive tube 102 remains stationary), the splined interface between the drive tube 102 and the piston rod 130 remains engaged, and the threaded interface between the piston rod 130 and the housing remains engaged.

Dispensing of a dose continues until the number display of the dose indicator 80 and the analog gauge return to their zero unit positions, when the zero unit abutment 81, 91 engages between the dose indicator 80 and the sliding gauge 90, or until the user releases the dispense button 30. When the zero unit abutment engages, torque from the motor spring 110 is reacted via the dose indicator 80 and sliding gauge 90 into the housing 10. When the user releases the dispense button 30, the action of the dispense spring 70 acts to return the axially displaced components to their proximal positions, which reengages the splined interface between the drive sleeve 101 and the housing, and reestablishes the connection between the dial grip 20 and the dose indicator 80, so that the user can modify the set dose as required.

As a preferred option, the device comprises a last dose stop including a last dose nut 50 which is threadedly engaged with the dial tube 40 and rotationally constrained to the clicker 60 via a splined interface. As the dial tube 40 is rotated to set a dose, the last dose nut 50 advances along the threaded path on the dial tube 40 towards a rotational abutment with stop surfaces 41 and 51 (Figure 10), occurring at the proximal end of the threaded path. When the last dose nut 50 reaches the proximal end of the threaded path, a rotational abutment is created by contact of surfaces 41 and 51 which rotationally couples the dial tube 40 to the clicker 60. As the clicker 60 is splined to the housing 10, the dial tube 40 becomes rotationally constrained and the user is unable to increase the set dose. The length of the threaded path on the dial tube 40 corresponds to the maximum number of doses that can be dispensed from the device. During dispensing, the dial tube 40 is rotationally constrained to the housing, via the splined engagement between the dispense button 30 and the proximal cap 12, occurring when the dispense button 30 is axially displaced. The last dose nut 50, dial tube 40 and clicker 60 are therefore held stationary, relative to the housing 10 during a dispense event.

During dose dispense, audible feedback is created by the interaction of the drive tube 102 and housing 10. As shown in Figure 12 a compliant clicker arm 103 is integrated into the drive tube 102 which is deflected as the drive tube 102 rotates inside a profiled surface in the main housing 11. The clicker arm 103 is positioned on the drive tube 102 aligned with the spline features on the inner bore which engage with the piston rod 130. The spline features on the piston rod 130 create clearance, enabling the clicker arm 103 to be deflected.

The profiled surface in the housing may have repetitive features corresponding to each delivered unit, which generate dispense 'clicks' for each dispensed unit. In an alternative embodiment the profiled surface may have repetitive features corresponding to a plurality of delivered units, which generate dispense 'clicks' when some multiple of units has been delivered. For example, this may be every second unit, or in another example this may be every sixth unit. Increasing the numbers of doses between successive dispense 'clicks' may be beneficial in a device with a high dispense rate.

At the completion of the delivery of a dose, as the dose indicator 80 returns to its zero unit orientation, and the sliding gauge 90 returns to its zero unit position, additional audible feedback is created by the interaction of the dose indicator 80, sliding gauge 90 and the clicker 60. This interaction is dependent on the axial position of the clicker 60, and only occurs during dispense, when the clicker 60 is in its distal position, when the dispense button 30 is depressed by the user. By utilizing the axial position of the dispense button 30 to create this interaction, the end of dose feature does not need to be overhauled by the user during dialing of a dose.

In the embodiment shown in Figures 13a to 14, a compliant end of dose clicker arm 82 is incorporated into the dose indicator 80, in a region of the dose indicator 80 which contains the threadform which engages with the thread on the sliding gauge 90. During dialing of a dose, this compliant end of dose clicker arm 82 is in its natural (unstressed) state as shown in Figure 13a, and is not disturbed by the motion of the sliding gauge 90 as it advances away from, or towards, its zero unit abutment. When the user depresses the dispense button 30, the clicker 60 is displaced distally. Displacement of the clicker 60, which is located internally to the dose indicator 80, creates an abutment between a distal face of the clicker 60 and a protrusion on the end of dose clicker arm 82, such that the end of dose clicker arm 82 is deflected distally as shown in Figure 13b. Deflection of the end of dose clicker arm 82 is independent of the orientation of the dose indicator 80.

When the dispense button 30 is depressed, the device will dispense medicament. The dose indicator 80 will rotate anti-clockwise (when viewed from the proximal end of the device) and the sliding gauge 90 will move distally. With the dispense button 30 depressed, movement of the dose indicator 80 and sliding gauge 90 in the opposing directions is not possible. As the sliding gauge 90 approaches its zero unit abutment with the dose indicator 80, the helical thread on the sliding gauge 90 engages with the deflected end of dose clicker arm 82. The tip of the end of dose clicker arm 82 is deformed in the proximal direction by the sliding gauge 90. This generates an increased axial contact force between the clicker 60 and the end of dose clicker arm 82, which via the direct load path to the dispense button 30 is felt by the user. The tip of the end of dose clicker arm 82 continues to be deformed proximally until the zero unit abutment position is reached, when a recess feature 93 (Figure 14) within the helical thread on the sliding gauge 90 releases the tip of the end of dose clicker arm 82, which returns to its deflected state. The release of the tip of the end of dose clicker arm 82 generates an impact with the recessed feature 93 which produces an audible 'click'. As the tip of the end of dose clicker arm 82 is released, the axial contact force between the clicker 60 and the end of dose clicker arm 82 is suddenly reduced, which via the direct load path to the dispense button 30 is felt by the user.

The user is therefore provided with audible and tactile feedback indicating that dispense of the dose has completed, and that the device has returned to the zero unit position.

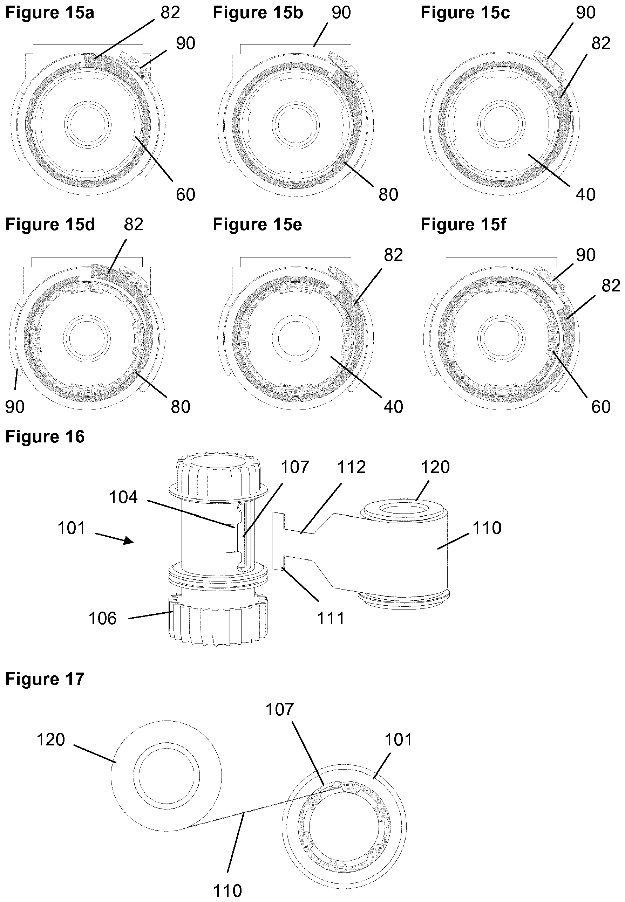

In an alternative embodiment shown in Figures 15a to 15f, a compliant end of dose clicker arm 82 is incorporated into the dose indicator 80 which is deflected radially by the displacement of the clicker 60 during dispense. A protrusion on the sliding gauge 90 deforms the deflected end of dose clicker arm 82 radially as the sliding gauge 90 approaches the zero unit abutment. When the zero unit position is reached, the protrusion on the sliding gauge 90 releases the deflected end of dose clicker arm 82, which may contact a further surface on the sliding gauge 90 or on the housing 10. In its natural state (Figures 15a to 15c), during dialing, the end of dose clicker arm 82 has a similar overall diameter to the main cylindrical surface of the dose indicator 80. The end of dose clicker arm 82 is therefore able to rotate freeing without contacting the protrusion on the distal end of the sliding gauge 90. This enables the user to dial up from the zero unit position without overhauling the end of dose clicker arm 82.

During dispensing, when the dispense button 30 is depressed, the clicker 60 is displaced distally. Its outer cylindrical surface contacts a boss on the inner surface of the end of dose clicker arm 82, causing the end of dose clicker arm 82 to be deflected radially (Figure 15d). This radial deflection increases the effective diameter at the tip of the end of dose clicker arm 82. The dose indicator 80 is free to rotate without any further interaction with the end of dose clicker arm 82 whilst the sliding gauge 90 is proximally displaced (i.e. at dose position > 5 units). As the sliding gauge 90 approaches its zero unit position and the dose indicator 80 approaches its zero unit orientation, the protrusion on the sliding gauge 90 engages with the deflected end of dose clicker arm 82. The tip of the end of dose clicker arm 82 is deformed radially by the contact with the protrusion on the sliding gauge 90 (Figure 15e). As the dose indicator 80 rotates to the zero unit position, the tip of the end of dose clicker arm 82 rotates past the protrusion, so that the end of dose clicker arm 82 is released and returns to its deflected state (Figure 15f). The tip of the end of dose clicker arm 82 can contact a further surface on the sliding gauge 90, or the housing to generate a 'click'. This embodiment minimizes the disturbance of the threadform surface of the dose indicator 80, which may be used as a visual element of the analog gauge feature of the device.

The motor spring 110 consists of a long strip of material, which has been rolled such that its natural state is to form a tightly wound spiral with a small inner diameter. The motor spring 110 is fitted onto the storage spool 120, which consists of a cylindrical surface with an outer diameter slightly larger than the natural inner diameter of the motor spring 110. There is no anchorage at the inner end of the motor spring 110 to the storage spool 120. The storage spool 120 may be a simple tube of material. Alternatively the storage spool may have a flange at one end, or it may have flanges at both ends. The flanges act to constrain and guide the material strip of the motor spring 110.

The outer end of the strip of material of the motor spring 110 may not be rolled, such that the end of the strip remains flat and does not wind itself onto the storage spool 120. The end of the strip of material of the motor spring 110 may have a profiled endform, to facilitate anchorage of the strip to features on the drive sleeve 101. As shown in Figure 16 the endform may consist of a portion 111 of wide length of strip at the extreme end of the strip, followed by a portion 112 of narrowed length of strip, before returning to the standard strip width. There may be a significant manufacturing tolerance associated with the length of the flat section of the strip on the end of the material of the motor spring 110, which must be accommodated during the assembly process.

The drive sleeve 101 consists of an outer cylindrical surface which forms the drive spool surface, onto which the motor spring 110 material is wound as the motor spring 110 is charged. Anchorage features are provided in the drive spool surface which engage with the endform of the strip of material of the motor spring 110. The anchorage features may include an axial slot 107 in the surface of the drive spool, which is significantly wider than the thickness of the strip. An axial slot 107 which is significantly wider than the thickness of the strip is advantageous during assembly of the motor spring 110 with the drive sleeve 101, as it can accommodate greater variation and tolerances in the position and alignment of the components.

The storage spool 120, with the motor spring 110 wound onto it, is moved towards the drive sleeve 101, so that the end of the material strip passes through the axial slot in the drive sleeve 101. Passing the material strip though the slot 107 in this orientation enables a larger tolerance in the length of the flat end of the strip to be accommodated, as subsequent assembly stages are insensitive to the amount by which the extreme end of the strip protrudes into the inner bore of the drive sleeve 101.

To engage the endform on the strip of material with the anchorage features in the drive sleeve 101, the drive sleeve 101 is rotated. The sense of rotation applied to the drive sleeve 101 to engage the anchorage feature is the same as the sense of rotation required to charge the motor spring 110. As the drive sleeve 101 is rotated, the strip of material is contacted by the radial faces of the axial slot 107 in the drive sleeve 101. This causes the strip of material to be deflected and the storage spool 120 to rotate. Rotation of the drive sleeve 101 continues until the strip of material becomes tangentially aligned to the cylindrical drive spool surface as shown in Figure 17. Once the strip material is tangential to the cylindrical surface, the endform on the strip material becomes fully engaged with the anchorage feature on the drive sleeve 101.

The anchorage feature on the drive sleeve 101 consists of a narrow recess 104 which matches the narrowed strip width 112 on the endform of the motor spring 110 material. Either side of this narrow recess 104, the cylindrical drive spool surface generates an abutment surface, with the wide length 111 of strip at the extreme end of the strip, created from a pocketed region on the inner cylindrical surface of the drive sleeve 101. When fully engaged, the extreme end 111 of the strip of material of the motor spring 110 occupies this pocketed region, such that a plain cylindrical bore of the drive sleeve 101 is maintained. Maintaining this plain cylindrical bore is advantageous as it provides a free running bearing surface with the dial tube 40.

Once the motor spring 110 endform is fully engaged with the drive sleeve 101, the drive sleeve 101 can be rotated a number of turns to charge the motor spring 110. Once charged, the majority of material of the motor spring 110 is wrapped around the drive sleeve 101. During dispensing of a dose, strip material is transferred back to the storage spool 120. When the last dose from the device has been dispensed, the majority of material of the motor spring 110 has been transferred back to the storage spool 120.

## Claims

1. A handheld injection device comprising
- a housing (10),
- a piston rod (130) located within the housing (10),
- a drive member (100),
- a power reservoir (110) for driving the drive member (100) comprising a reverse wound flat spiral spring as a power reservoir having a first end attached to a first spool (120) and a second end attached to a second spool,
**characterized in that** the drive member (100) is permanently coupled to the piston rod (130), wherein the drive member (100) is axially movable between a dose setting position, in which the drive member (100) is rotationally constrained to the housing (10), and a dose dispensing position, in which the drive member (100) is rotationally de-coupled from the housing (10), and wherein the second spool is axially and rotationally constrained to drive member (100).

2. The injection device according to claim 1, wherein the second end of the spring (110) comprises a portion (112) of reduced width and a free end portion (111) having an increased width compared with the portion (112) of reduced width, and wherein the drive member (100) comprises a cylindrical spool portion having an axial slot (107) and an adjacent narrow recess (104).

3. The injection device according to claims 1 or 2, further comprising a dose indicator (80), which is axially constrained to the housing (10) and which during dose setting rotates relative to the housing (10) in either a first direction or a second opposite direction and which during dose dispensing rotates relative to the housing (10) in the second opposite direction.

4. The injection device according to claim 3, further comprising a gauge element (90), which is at least partly interposed between the housing (10) and the dose indicator (80) and at least partly visible through at least one aperture or window of the housing (10), wherein the gauge element (90) is axially guided within the housing (10) and in threaded engagement with the dose indicator (80) such that rotation of the dose indicator (80) causes an axial displacement of the gauge element (90).

5. The injection device according to claims 3 and 4, further comprising a limiter mechanism (81; 91, 92) defining a maximum settable dose and a minimum settable dose, which limiter mechanism comprises a first rotational stop (81) on the dose indicator (80) and a first counter stop (91) on the gauge element (90), which abut in the minimum dose (zero) position, and a second rotational stop on the dose indicator (80) and a second counter stop (92) on the gauge element (90), which abut in the maximum dose position.

6. The injection device according to claims 3 and 4, wherein the housing (10) has an aperture or window and the gauge element (90) has a further aperture or window, which is positioned with respect to the aperture or window of the housing (10) such that at least a part of the dose indicator (80) is visible through the apertures or windows.

7. The injection device according to any of the preceding claims, further comprising an axially displaceable dispense button (30) and a dial grip (20) which is axially constrained to the housing (10).

8. The injection device according to claim 7, further comprising a clicker sleeve (60) which is rotationally constrained to the housing (10) and is axially displaceable relative to the housing (10) between a proximal dose setting position and a distal dose dispensing position, wherein the clicker sleeve (60) comprises teeth releasably engaging corresponding teeth of a further element (40) which is rotatable during dose setting.

9. The injection device according to claim 7, wherein the dose indicator (80) comprises a flexible clicker arm (82), which is displaceable by the clicker sleeve (60) in a first direction and only during dose dispensing when the device reaches its minimum dose (zero) position in a second, opposite direction by a protruding section of the gauge element (90).

10. The injection device according to claims 7 or 8, further comprising a last dose protection mechanism (40, 50, 60) for preventing the setting of a dose, which exceeds the amount of liquid left in a cartridge, which last dose protection mechanism comprises a nut member (50) located interposed between the clicker sleeve (60) and a dial sleeve (40).

11. The injection device according to any of the preceding claims, wherein first spool (120) is located concentrically with the piston rod (130) on a first longitudinal axis (I), and the second spool is located on a second longitudinal axis (II), wherein the first longitudinal axis (I) is parallel to and spaced from the second longitudinal axis (II).

12. The injection device according to claim 11, wherein the drive member (100) comprises a drive tube (102) which is rotatable about the first longitudinal axis (I) and a drive sleeve (101) which is rotatable about the second longitudinal axis (II).

13. The injection device according to claim 12, wherein the drive sleeve (101) is axially movable between the dose setting position, in which the drive sleeve (101) is rotationally constrained to the housing (10), and the dose dispensing position, in which the drive sleeve (101) is rotationally de-coupled from the housing (10), and wherein the drive tube (102) is permanently rotationally coupled to the drive sleeve (101).

14. The injection device according to any of the preceding claims, further comprising a clutch (83, 105) provided interposed between the dose indicator (80) and the drive member (100), wherein the clutch (83, 105) allows relative rotational movement between the dose indicator (80) and the drive member (100) during dose setting and prevents relative rotational movement between the dose indicator (80) and the drive member (100) during dose dispensing.

15. The injection device according to any of the preceding claims comprising a cartridge containing a medicament.

## Patentansprüche

1. Handgehaltene Injektionsvorrichtung, umfassend
- ein Gehäuse (10),
- eine Kolbenstange (130), die im Gehäuse (10) angeordnet ist,
- ein Antriebselement (100),
- einen Energiespeicher (110) zum Antreiben des Antriebselements (100), umfassend eine umgekehrt gewickelte flache Spiralfeder als Energiespeicher, deren erstes Ende an einer ersten Spule (120) befestigt ist und deren zweites Ende an einer zweiten Spule befestigt ist,
**dadurch gekennzeichnet, dass** das Antriebselement (100) permanent mit der Kolbenstange (130) verbunden ist, wobei das Antriebselement (100) zwischen einer Dosiseinstellposition, in der das Antriebselement (100) drehfest am Gehäuse (10) zurückgehalten wird, und einer Dosisabgabeposition, in der das Antriebselement (100) drehbar vom Gehäuse (10) gelöst ist, axial bewegbar ist, und wobei die zweite Spule axial und drehfest am Antriebselement (100) zurückgehalten wird.

2. Injektionsvorrichtung nach Anspruch 1, wobei das zweite Ende der Feder (110) einen Abschnitt (112) mit reduzierter Breite und einen freien Endabschnitt (111) mit erhöhter Breite im Vergleich zum Abschnitt (112) mit reduzierter Breite umfasst und wobei das Antriebselement (100) einen zylinderischen Spulenabschnitt mit einem axialen Schlitz (107) und einer benachbarten schmalen Aussparung (104) umfasst.

3. Injektionsvorrichtung nach Anspruch 1 oder 2, ferner umfassend einen Dosisanzeiger (80), der axial am Gehäuse (10) zurückgehalten wird und der sich während der Dosiseinstellung bezüglich des Gehäuses (10) entweder in eine erste Richtung oder in eine zweite, entgegengesetzte Richtung dreht und der sich während der Dosisabgabe bezüglich des Gehäuses (10) in die zweite, entgegengesetzte Richtung dreht.

4. Injektionsvorrichtung nach Anspruch 3, ferner umfassend ein Messelement (90), das zumindest teilweise zwischen dem Gehäuse (10) und dem Dosisanzeiger (80) angeordnet ist und das zumindest teilweise durch mindestens eine Öffnung oder ein Fenster des Gehäuses (10) sichtbar ist, wobei das Messelement (90) axial im Gehäuse (10) geführt wird und mit dem Dosisanzeiger (80) in Gewindeeingriff steht, derart, dass eine Drehung des Dosisanzeigers (80) eine axiale Verschiebung des Messelements (90) verursacht.

5. Injektionsvorrichtung nach den Ansprüchen 3 und 4, ferner umfassend einen Begrenzungsmechanismus (81; 91, 92), der eine einstellbare Höchstdosis und eine einstellbare Mindestdosis definiert, wobei der Begrenzungsmechanismus eine erste Drehsperre (81) auf dem Dosisanzeiger (80) und eine erste Gegensperre (91) auf dem Messelement (90), die in der Position der Mindestdosis (null) aneinanderstoßen, und eine zweite Drehsperre auf dem Dosisanzeiger (80) und eine zweite Gegensperre (92) auf dem Messelement (90), die in der Position der Höchstdosis aneinanderstoßen, umfasst.

6. Injektionsvorrichtung nach den Ansprüchen 3 und 4, wobei das Gehäuse (10) eine Öffnung oder ein Fenster aufweist und das Messelement (90) eine weitere Öffnung oder ein Fenster aufweist, die bzw. das bezüglich der Öffnung oder des Fensters des Gehäuses (10) derart angeordnet ist, dass zumindest ein Teil des Dosisanzeigers (80) durch die Öffnungen oder Fenster sichtbar ist.

7. Injektionsvorrichtung nach einem der vorhergehenden Ansprüche, ferner umfassend einen axial verschiebbaren Ausgabeknopf (30) und einen Wählgriff (20), der axial am Gehäuse (10) zurückgehalten wird.

8. Injektionsvorrichtung nach Anspruch 7, ferner umfassend eine Klickerhülse (60), die drehfest am Gehäuse (10) zurückgehalten wird und bezüglich des Gehäuses (10) zwischen einer proximalen Dasiseinstellposifiion und einer distalen Dosisabgabeposition axial verschiebbar ist, wobei die Klickerhülse (60) Zähne umfasst, die lösbar in entsprechende Zähne eines weiteren Elements (40), das während der Dosiseinstellung drehbar ist, eingreifen,

9. Injektionsvorrichtung nach Anspruch 7, wobei der Dosisanzeiger (80) einen flexiblen Klickerarm (82) umfasst, der von der Klickerhülse (60) in eine erste Richtung verschoben werden kann und nur während der Dosisabgabe, wenn die Vorrichtung ihre Position der Mindestdosis (null) erreicht, von einem vorragenden Abschnitt des Messelements (90) in eine zweite, entgegengesetzte Richtung verschoben werden kann.

10. Injektionsvorrichtung nach den Ansprüchen 7 oder 8, ferner umfassend einen letzten Dosisschutzmechanismus (40, 50, 60) zur Verhinderung des Einstellens einer Dosis, die die in einer Kartusche verbleibende Flüssigkeitsmenge überschreitet, wobei der letzte Dosisschutzmechanismus ein Mutternelement (50) umfasst, das zwischen der Klickerhülse (60) und einer Wahlhülse (40) angeordnet ist.

11. Injektionsvorrichtung nach einem der vorhergehenden Ansprüche, wobei die erste Spule (120) konzentrisch mit der Kolbenstange (130) auf einer ersten Längsachse (I) angeordnet ist und die zweite Spule auf einer zweiten Längsachse (II) angeordnet ist, wobei die erste Längsachse (I) parallel zur zweiten Längsachse (II) und von dieser beabstandet ist.

12. Injektionsvorrichtung nach Anspruch 11, wobei das Antriebselement (100) eine um die erste Längsachse (I) drehbare Antriebsröhre (102) und eine um die zweite Längsachse (II) drehbare Antriebshülse (101) umfasst.

13. Injektionsvorrichtung nach Anspruch 12, wobei die Antriebshülse (101) zwischen der Dosiseinstellposition, in der die Antriebshülse (101) drehfest am Gehäuse (10) zurückgehalten wird, und der Dosisabgabeposition, in der die Antriebshülse (101) drehbar vom Gehäuse (10) gelöst ist, axial bewegbar ist und wobei die Antriebsröhre (102) permanent drehbar mit der Antriebshülse (101) verbunden ist.

14. Injektionsvorrichtung nach einem der vorhergehenden Ansprüche, ferner umfassend eine Kupplung (83, 105), die zwischen dem Dosisanzeiger (80) und dem Antriebselement (100) angeordnet ist, wobei die Kupplung (83, 105) eine relative Drehbewegung zwischen dem Dosisanzeiger (80) und dem Antriebselement (100) während der Dosiseinstellung gestattet und eine relative Drehbewegung zwischen dem Dosisanzeiger (80) und dem Antriebselement (100) während der Dosisabgabe verhindert.

15. Injektionsvorrichtung nach einem der vorhergehenden Ansprüche, umfassend eine Kartusche, die ein Medikament enthält.

## Revendications

1. Dispositif d'injection portatif comprenant :
- un boîtier (10),
- une tige de piston (130) située dans le boîtier (10),
- un élément d'entraînement (100),
- un réservoir d'énergie (110) pour entraîner l'élément d'entraînement (100) comprenant un ressort hélicoïdal plat à enroulement inverse en tant que réservoir d'énergie ayant une première extrémité fixée à une première bobine (120) et une seconde extrémité fixée à une seconde bobine,
**caractérisé en ce que** l'élément d'entraînement (100) est couplé de manière permanente à la tige de piston (130), dans lequel l'élément d'entraînement (100) est axialement mobile entre une position de réglage de dose, dans laquelle l'élément d'entraînement (100) est contraint en rotation par rapport au boîtier (10), et une position de distribution de dose, dans laquelle l'élément d'entraînement (100) est désolidarisé en rotation du boîtier (10), et dans lequel la seconde bobine est contrainte axialement et en rotation par rapport à l'élément d'entraînement (100).

2. Dispositif d'injection selon la revendication 1, dans lequel la seconde extrémité du ressort (110) comprend une partie (112) de largeur réduite et une partie d'extrémité libre (111) ayant une largeur accrue par comparaison avec la partie (112) de largeur réduite et dans lequel l'élément d'entraînement (100) comprend une partie de bobine cylindrique ayant une fente axiale (107) et un évidement étroit adjacent (104).

3. Dispositif d'injection selon les revendications 1 ou 2, comprenant en outre un indicateur de dose (80), qui est axialement contraint par rapport au boîtier (10) et qui, pendant un réglage de dose, tourne par rapport au boîtier (10) sois dans une première direction, soit dans une seconde direction opposée et qui, pendant une distribution de dose, tourne par rapport au boîtier (10) dans la seconde direction opposée.

4. Dispositif d'injection selon la revendication 3, comprenant en outre un élément de jauge (90), qui est au moins partiellement intercalé entre le boîtier (10) et l'indicateur de dose (80) et au moins partiellement visible à travers au moins une ouverture, ou une fenêtre, du boîtier (10), dans lequel l'élément de jauge (90) est axialement guidé dans le boîtier (10) et est en contact de manière filetée avec l'indicateur de dose (80) de telle sorte que la rotation de l'indicateur de dose (80) provoque un déplacement axial de l'élément de jauge (90).

5. Dispositif d'injection selon les revendications 3 et 4, comprenant en outre un mécanisme de limitation (81 ; 91, 92) définissant une dose réglable maximale et une dose réglable minimale, lequel mécanisme de limitation comprend une première butée de rotation (81) sur l'indicateur de dose (80) et une première contre-butée (91) sur l'élément de jauge (90), qui viennent en butée dans la position de dose minimale (nulle), et un second arrêt de rotation sur l'indicateur de dose (80) et une seconde contre-butée (92) sur l'élément de jauge (90), qui viennent en butée dans la position de dose maximale.

6. Dispositif d'injection selon les revendications 3 et 4, dans lequel le boîtier (10) comporte une ouverture, ou une fenêtre, et l'élément de jauge (90) comporte une autre ouverture, ou une autre fenêtre, qui est positionnée par rapport à l'ouverture, à la fenêtre, du boîtier (10) de telle sorte qu'au moins une partie de l'indicateur de dose (80) soit visible à travers les ouvertures ou les fenêtres.

7. Dispositif d'injection selon l'une quelconque des revendications précédentes, comprenant en outre un bouton de distribution pouvant être déplacé axialement (30) et une molette (20) qui est contrainte axialement par rapport au boîtier (10).

8. Dispositif d'injection selon la revendication 7, comprenant en outre un manchon à cliquet (60) qui est contraint en rotation par rapport au boîtier (10) et peut être déplacé axialement par rapport au boîtier (10) entre une position de réglage de dose proximale et une position de distribution de dose distale mais, dans lequel le manchon à cliquet (60) comprend des dents qui s'engrènent de manière amovible avec des dents correspondantes d'un autre élément (40) qui peut être tourné pendant un réglage de dose.

9. Dispositif d'injection selon la revendication 7, dans lequel l'indicateur de dose (80) comprend un bras à cliquet flexible (82), qui peut être déplacé par le manchon à cliquet (60) dans une première direction et pendant seulement une distribution de dose lorsque le dispositif atteint sa position de dose minimale (nulle) dans une seconde direction opposée par une section saillante de l'élément de jauge (90).

10. Dispositif d'injection selon les revendications 7 ou 8, comprenant en outre un dernier mécanisme de protection (40, 50, 60) pour empêcher le réglage d'une dose, qui dépasse la quantité de liquide laissée dans une cartouche, lequel dernier mécanisme de protection de dose comprend un élément d'écrou (50) placé de façon à être intercalé entre le manchon à cliquet (60) et une molette (40).

11. Dispositif d'injection selon l'une quelconque des revendications précédentes, une première bobine (120) est placée de manière concentrique avec la tige de piston (130) sur un premier axe longitudinal (I) et la seconde bobine est située sur un second axe longitudinal (II), dans lequel le premier axe longitudinal (I) est parallèle au second axe longitudinal (II) et espacé de ce dernier.

12. Dispositif d'injection selon la revendication 11, dans lequel l'élément d'entraînement (100) comprend un tube d'entraînement (102) qui peut tourner autour du premier axe longitudinal (I), et un manchon d'entraînement (101) qui peut tourner autour du second axe longitudinal (II).

13. Dispositif d'injection selon la revendication 12, dans lequel le manchon d'entraînement (101) peut se déplacer axialement entre la position de réglage de dose, dans laquelle le manchon d'entraînement (101) est contraint en rotation par rapport au boîtier (10), et la position de distribution de dose dans laquelle le manchon d'entraînement (101) est désolidarisé en rotation du boîtier (10), et dans lequel le tube d'entraînement (102) est couplé en rotation de façon permanente au manchon d'entraînement (101).

14. Dispositif d'injection selon l'une quelconque des revendications précédentes, comprenant en outre un embrayage (83, 105) disposé de façon à être intercalé entre l'indicateur de dose (80) et l'élément d'entraînement (100), dans lequel l'embrayage (83, 105) permet un mouvement de rotation relatif entre l'indicateur de dose (80) et l'élément d'entraînement (100) pendant un réglage de dose et empêche un mouvement de rotation relatif entre l'indicateur de dose (80) et l'élément d'entraînement (100) pendant une distribution de dose.

15. Dispositif d'injection selon l'une quelconque des revendications précédentes, comprenant une cartouche contenant un médicament.
